Europäisches Patentamt

**European Patent Office**

Office européen des brevets

(19)

(11)     **EP 1 066 819 B1**

(12)     **FASCICULE DE BREVET EUROPEEN**

(45) Date de publication et mention
de la délivrance du brevet:
**07.12.2005   Bulletin 2005/49**

(51) Int Cl.$^7$: **A61K 7/032**

(21) Numéro de dépôt: **00401398.3**

(22) Date de dépôt: **19.05.2000**

(54) **Mascara comprenant une dispersion de particules de polymère dans une phase grasse liquide**

Mascara die eine Dispersion von Polymerteilchen in einer flüssigen Fettphase enthält

Mascara comprising a dispersion of polymer particles in a liquid oil phase

(84) Etats contractants désignés:
**AT BE CH CY DE DK ES FI FR GB GR IE IT LI LU
MC NL PT SE**

(30) Priorité: **21.06.1999   FR 9908056**

(43) Date de publication de la demande:
**10.01.2001   Bulletin 2001/02**

(73) Titulaire: **L'OREAL
75008 Paris (FR)**

(72) Inventeurs:
• **De La Poterie, Valérie
77820 Le Chatelet-en-Brie (FR)**

• **Piot, Bertrand
75009 Paris (FR)**

(74) Mandataire: **Kromer, Christophe
L'OREAL - D.I.P.I.
25-29 Quai Aulagnier
92600 Asnières (FR)**

(56) Documents cités:
**EP-A- 0 497 144          EP-A- 0 749 746
EP-A- 0 749 747          EP-A- 0 987 012
EP-A- 1 002 528          WO-A-95/15741
WO-A-98/42298**

**Description**

La présente invention concerne un mascara comprenant des particules de polymère dispersées dans une phase grasse liquide. L'invention se rapporte également à l'utilisation de cette composition pour le maquillage des fibres kératiniques, ainsi qu'à un procédé de maquillage de ces dernières. Le mascara et le procédé de maquillage selon l'invention sont plus particulièrement destinés aux fibres kératiniques sensiblement longitudinales telles que les cils, les sourcils et les cheveux d'être humains, y compris les faux-cils et les postiches. La composition peut être une composition de maquillage, une base de maquillage, une composition à appliquer sur un maquillage, dite encore top-coat, ou bien encore une composition de traitement cosmétique des fibres kératiniques. Plus spécialement, l'invention porte sur un mascara.

Les compositions de revêtement des cils, appelées mascara, comprennent généralement, de façon connue, au moins une cire et un polymère filmogène pour déposer un film coloré sur les cils et gainer ces derniers, comme le décrit par exemple le document WO-A-95/15741. Il est aussi connu du document EP-A-749746 une composition de mascara comprenant une faible quantité de polymère dispersé dans de l'isoparaffine.

Toutefois, ces compositions ne permettent pas d'obtenir un film résistant aux frottements par exemple des doigts ou des tissus (mouchoirs, serviettes) et le film se désagrège en partie en s'effritant ou bien encore en s'étalant. L'effritement du film engendre une perte sensible de l'intensité de la couleur du maquillage, obligeant ainsi la consommatrice à renouveler l'application du produit de maquillage. L'étalement du film forme, quant à lui, une auréole autour de la zone maquillée très inesthétique. L'eau, notamment l'humidité de l'atmosphère par temps de pluie, les larmes et la transpiration accentuent ces inconvénients, de même que le sébum des peaux à tendance grasse. Le film de maquillage ne présente plus globalement une bonne tenue dans le temps.

Le but de la présente invention est de proposer un mascara présentant pas les inconvénients ci-dessus et conduisant, après application sur les fibres kératiniques, à la formation d'un film ayant une bonne tenue dans le temps et résistant aux frottements, notamment des doigts ou des tissus, à sec et/ou à l'eau et/ou au sébum et/ou à la transpiration.

Les inventeurs ont découvert qu'un tel mascara pouvait être obtenu en utilisant une quantité efficace d'un polymère dispersé dans une phase grasse liquide.

Plus précisément, l'invention a pour objet un mascara comprenant dans un milieu physiologiquement acceptable, une dispersion de particules de polymère stabilisées en surface dans une phase grasse liquide, caractérisée par le fait qu'elle comprend au moins 2 % en poids, par rapport au poids total de la composition, desdites particules de polymère au moins une matière colorante, et que la composition a une viscosité, mesurée à 25 °C, à un cisaillement de 200 s$^{-1}$, allant de 2 Pa.s à 17 Pa.s.

L'invention a aussi pour objet un procédé de revêtement des fibres kératiniques, notamment des cils, consistant à appliquer sur les fibres kératiniques une composition telle que décrite précédemment.

L'invention a également pour objet l'utilisation d'un mascara tel que défini précédemment pour obtenir un film déposé sur les fibres kératiniques résistant aux frottements.

Le mascara selon l'invention a une viscosité allant de 2 Pa.s à 17 Pa.s (20 à 170 poises), et de préférence de 5 Pa.s à 13 Pa.s (50 à 130 poises) pour permettre une application aisée de la composition sur les fibres kératiniques, notamment les cils.

La viscosité est mesurée à 25 °C, avec un viscosimètre RHEOMATT 180 vendu par la société RHEO, à une vitesse de cisaillement de 200 s$^{-1}$, la mesure étant effectuée après 10 minutes de rotation (temps au bout duquel on observe une stabilisation de la viscosité et de la vitesse de rotation du mobile).

La mesure de viscosité est effectuée avec le mobile n°2 lorsque la viscosité va de 20 centipoises à 7,32 poises (0,02 à 0,732 Pa.s) ; avec le mobile n°3 pour une viscosité allant de 1,8 poise à 40,2 poises (0,18 à 4,02 Pa.s) ; avec le mobile n° 4 pour une viscosité allant de 12 poises à 236 poises (1,2 à 23,6 Pa.s) ; avec le mobile n° 5 pour une viscosité allant de 80 poises à 1220 poises (8 à 122 Pa.s).

Le mascara selon l'invention comprend une dispersion stable de particules généralement sphériques d'au moins un polymère, dans une phase grasse liquide physiologiquement acceptable. Ces dispersions peuvent notamment se présenter sous forme de nanoparticules de polymères en dispersion stable dans ladite phase grasse. Les nanoparticules ont de préférence une taille allant de 5 à 600 nm, étant donné qu'au-delà d'environ 600 nm, les dispersions de particules deviennent beaucoup moins stables, et de préférence de 50 nm à 250 nm.

Le polymère utilisé dans la présente invention peut être de toute nature. On peut ainsi employer un polymère radicalaire, un polycondensat, voire un polymère d'origine naturelle et leurs mélanges. Le polymère peut être choisi par l'homme du métier en fonction de ses propriétés.

L'un des avantages de la dispersion de polymère de la composition de l'invention est la possibilité de faire varier la température de transition vitreuse (Tg) du polymère ou du système polymérique (polymère plus additif du type plastifiant), et de passer ainsi d'un polymère mou à un polymère plus ou moins dur, permettant de régler les propriétés mécaniques du film obtenu avec la composition de l'invention.

Les polymères utilisables dans la composition de l'invention ont de préférence un poids moléculaire moyen en poids allant de l'ordre de 2000 à 10 000 000. Le polymère peut avoir une température de transition vitreuse allant de -100°C à 300°C, et mieux de -10 °C à 50 °C.

Il est possible d'utiliser des polymères filmifiables, de préférence ayant une température de transition vitreuse basse, inférieure ou égale à la température de la peau, et notamment inférieure ou égale à environ 40 °C. On entend par "polymère filmifiable" un polymère apte à former à lui seul, ou en présence d'un agent plastifiant, un film isolable. On obtient ainsi une dispersion qui peut filmifier lorsqu'elle est appliquée sur un support, ce qui n'est pas le cas lorsque l'on utilise des dispersions de pigments minéraux selon l'art antérieur.

Lorsque le polymère présente une température de transition vitreuse trop élevée pour l'application souhaitée, on peut lui associer un plastifiant de manière à abaisser cette température du mélange utilisé. Le plastifiant peut être choisi parmi les plastifiants usuellement utilisés dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure ou égale à environ 40°C et notamment allant de - 10 °C à 30 °C, et leurs mélanges.

Parmi les polymères non filmifiables, on peut citer des homopolymères ou copolymères radicalaires, vinyliques ou acryliques, ayant de préférence une Tg supérieure à environ 40°C, et notamment allant de 45 °C à 100 °C, et leurs mélanges. Le polymère non filmifiable permet, en association avec la phase grasse liquide, de former un dépôt continu et homogène sur les cils.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés $\alpha,\beta$-éthyléniques tes que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{20}$, de préférence en $C_1$-$C_8$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le (méth)acrylate de méthyle, le (méth)acrylate d'éthyle, le (méth)acrylate de butyle, le (méth)acrylate d'isobutyle, le (méth)acrylate d'éthyl-2 hexyle, le (méth)acrylate de lauryle.

Parmi les (méth)acrylates d'hydroxyalkyle, on peut citer le (méth)acrylate d'hydroxyéthyle, le (méth)acrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Comme polymère radicalaire, on utilise de préférence les copolymère d'acide (méth)acrylique et de (méth) acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth) acrylamides, en particulier d'alkyl en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères vinyliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyl, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle.

Comme monomères styréniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques, on peut encore citer :

- la N-vinylpyrrolidone; la vinylcaprolactame; les vinyl N-alkyl($C_1$-$C_6$) pyrroles; les vinyl-oxazoles; les vinyl-thiazoles; les vinylpyrimidines; les vinylimidazoles;
- les oléfines tels que l'éthylène, le propylène, le butylène, l'isoprène, le butadiène.

Le polymère vinylique peut être réticulé à l'aide de monomère difonctionnel, notamment comprenant au moins deux insaturations éthyléniques, tel que le diméthacrylate d'éthylène glycol, le phtalate de diallyle

De façon non limitative, les polymères de l'invention peuvent être choisis parmi, les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; copolymères acryliques-silicone ; polyacrylamides ; polymères siliconés, polymères fluorés et leurs mélanges.

Avec une telle dispersion de particules de polymère, il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau ou les lèvres. Ceci ne serait pas possible avec des pigments sous forme particulaire, leur constitution ne permettant pas de moduler la taille moyenne des particules.

Le polymère dispersé est utilisé en une quantité efficace pour obtenir un film déposé sur les fibres kératiniques résistant aux frottements éventuellement en présence d'eau et/ou de sébum et/ou de transpiration.

En pratique, le polymère dispersé dans la phase grasse liquide peut être présent en une teneur allant de 2 % à 50 % en poids, par rapport au poids total de la composition, de préférence de 4 % à 40 % en poids, et mieux de 5 % à 30 % en poids.

Par "phase grasse liquide", on entend tout milieu non aqueux liquide à température ambiante (25 °C) et pression atmosphérique. Cette phase grasse peut contenir une phase grasse liquide volatile et/ou une phase grasse liquide non volatile contenant respectivement une ou plusieurs huiles liquides.

Par "phase grasse volatile", on entend tout milieu non aqueux susceptible de s'évaporer de la peau en moins d'une heure. Cette phase volatile comporte notamment des huiles ayant une pression de vapeur, à température ambiante et pression atmosphérique allant de $10^{-3}$ à 300 mm de Hg (0,13 Pa à 40 000 Pa).

La phase grasse liquide dans laquelle est dispersé le polymère, peut être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

La phase grasse liquide totale de la composition peut représenter de 5 % à 98 % en poids, par rapport au poids total de la composition et de préférence de 20 à 85 % en poids. La partie non volatile peut représenter de 0 à 80 % (notamment de 0,1 à 80 %) du poids total de la composition et mieux de 1 à 50%.

Comme phase grasse liquide utilisable dans l'invention, on peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxane (PDMS), éventuellement phénylées telles que les phényltriméthicones, ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

Avantageusement, on peut utiliser une ou plusieurs huiles volatiles à température ambiante. Ces huiles volatiles sont favorables à l'obtention d'un film à propriétés "sans transfert" total, à savoir résistant totalement aux frottements. Après évaporation de ces huiles, on obtient un dépôt filmogène souple, non collant. Ces huiles volatiles facilitent, en outre, l'application de la composition sur les fibres kératiniques comme les cils.

Ces huiles volatiles peuvent être des huiles hydrocarbonées, des huiles siliconées comportant éventuellement des groupements alkyle ou alkoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone, on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.

Comme huile hydrocarbonée volatile, on peut citer les isoparaffines en $C_8$-$C_{16}$ telles que les 'ISOPARs', les PERMETYLs et notamment l'isododécane.

Ces huiles volatiles peuvent être présentes dans la composition en une teneur allant de 5 à 97,99 % du poids total de la composition, et mieux de 30 à 75 %.

Dans un mode particulier de réalisation de l'invention, on choisit le phase grasse liquide dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

Le paramètre de solubilité global $\delta$ global selon l'espace de solubilité de HANSEN est défini dans l'article "Solubility parameter values" de Eric A. Grulke de l'ouvrage "Polymer Handbook" 3ème édition, Chapitre VII, pages 519-559 par la relation :

$$\delta = (d_D{}^2 + d_P{}^2 + d_H{}^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est décrite dans l'article de C. M. HANSEN : "The three dimensional solubility parameters" J. Paint Technol. 39, 105 (1967).

Parmi les phases grasses liquides ayant un paramètre de solubilité global selon l'espace de solubilité de HANSEN inférieur ou égal à 17 $(MPa)^{1/2}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les hydrocarbures et notamment des huiles de paraffine, de vaseline, ou le polyisobutylène hydrogéné, l'isododécane, ou encore les 'ISOPARs', isoparaffines volatiles. On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyle, thiol et/ou amine, et les huiles siliconées volatiles, notamment cycliques. On peut également citer les solvants, seuls ou en mélange, choisis parmi (i) les esters linéaires, ramifiés ou cycliques, ayant plus de 6 atomes de carbone, (ii) les éthers ayant plus de 6 atomes de carbone, (iii) les cétones ayant plus de 6 atomes de carbone. Par monoalcools ayant un paramètre de solubilité global selon l'espace de solubilité de HANSEN inférieur ou égal à 20 $(MPa)^{1/2}$, on entend les alcools gras aliphatiques ayant au moins 6 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Comme milieu non aqueux, on peut aussi utiliser ceux décrits dans le document FR-A-2 710 646 de L.V.M.H.

Le choix du milieu non aqueux est effectué par l'homme du métier en fonction de la nature des monomères constituant le polymère et/ou de la nature du stabilisant, comme indiqué ci-après.

La dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertio-butylperoxy-2-éthyl hexanoate.

Les particules de polymère sont stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

Le stabilisant peut aussi être choisi parmi les copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou encore les alkyl ($C_2$-$C_{18}$) méthicones copolyol. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200 par la société "DOW CORNING".

Comme copolymères blocs greffés ou séquencés, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly-(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylènelpolybutadiène ou polyoxyéthylène/polyisobutylène.

On peut également employer des copolymères de (méth)acrylates d'alkyl en $C_1$-$C_4$, et de (méth)acrylates d'alkyl en $C_8$-$C_{30}$. On peut en particulier citer le copolymère méthacrylate de stéaryle/méthacrylate de méthyle.

Lorsqu'on utilise un polymère statistique en tant que stabilisant, on le choisit de manière à ce qu'il possède une quantité suffisante de groupements le rendant soluble dans le solvant de synthèse envisagé.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxy ($C_2$-$C_{18}$)alkylène et notammment polyoxypropyléné et/ou oxyéthyléné.

Lorsque la phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyl en $C_1$-$C_4$, et d'acrylates ou de méthacrylates d'alkyl en $C_8$-$C_{30}$,
- (c) les copolymères blocs greffés ou séquencés comprenant au moins un bloc résultant de la polymérisation d'au moins un monomère éthylénique, à liaisons éthyléniques conjuguées,

et au moins un bloc d'un polymère vinylique ou acrylique ou d'un polyéther ou d'un polyester, ou leurs mélanges.

De préférence, on utilise des polymères dibloc comme agent stabilisant.

Suivant l'application, on pourra choisir d'utiliser des dispersions de polymères filmifiables ou non filmifiables, dans des huiles volatiles ou non volatiles.

La composition selon l'invention peut contenir, en outre, au moins une cire qui peut être choisie parmi les cires d'origine animale, les cires d'origine végétale ou les cires d'origine synthétique.

Les cires susceptibles d'être utilisées dans la composition selon l'invention possèdent en règle générale un point de fusion compris entre 40 et 110 °C . La cire peut également avoir une pénétrabilité à l'aiguille allant de 1 à 217. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Parmi les cires d'origine animale, on peut citer les cires d'abeille, les cires de lanoline et les cires d'insectes de Chine.

Parmi les cires d'origine végétale, on peut citer les cires de riz, les cires de Carnauba, de Candellila, d'Ouricurry, les cires de fibres de liège, les cires de canne à sucre, les cires du Japon, la cire de Sumac, la cire de coton.

Parmi les cires d'origine minérale, on peut citer les paraffines, les cires microcristallines, les cires de Montan et les ozokérites.

Parmi les cires d'origine synthétique, on peut utiliser notamment les cires de polyoléfine et notamment les cires de polyéthylène, les cires obtenues par la synthèse de Fischer et Tropsch, les copolymères cireux ainsi que leurs esters, les cires de silicone.

Il est également possible d'utiliser des huiles d'origine animale ou végétale hydrogénées qui répondent toujours aux deux caractéristiques physiques mentionnées précédemment.

Parmi ces huiles, on peut citer les huiles hydrogénées qui sont obtenues par hydrogénation catalytique de corps gras composés de chaîne grasse linéaire ou non en $C_8$-$C_{32}$, l'huile de tournesol hydrogénée, l'huile de ricin hydrogénée, l'huile de coprah hydrogénée, l'huile de jojoba hydrogénée, la lanoline hydrogénée et les huiles de palme hydrogénées. Les cires utilisables selon la présente invention sont de préférence solides et rigides à température inférieure à 50 °C.

La composition selon l'invention peut comprendre de 0 % à 30 % (notamment de 0,1 % à 30 %) en poids de cire, en poids par rapport au poids total de la composition, de préférence de 1 % à 25 % en poids.

De préférence, la composition selon l'invention peut comprendre :

- au moins une cire ayant une pénétrabilité à l'aiguille allant de 1 à 7, 5 (dite cire I), notamment en une teneur allant de 0,1 % à 20 % en poids par rapport au poids total de la composition, et
- au moins une cire ayant une pénétrabilité à l'aiguille supérieure à 7,5 et inférieure ou égale à 217 (dite cire II), notamment en une teneur allant de 0,1 % à 10 % en poids par rapport au poids total de la composition.

La composition peut aussi contenir un polymère solubilisé dans la phase grasse liquide, dit encore polymère liposoluble.

Comme polymère lipophile, on peut notamment coter les copolymères résulatant de la copolyméisation d'au moins un ester vinylique et d'au moins un autre monomère qui peut être une -oléfine, un alkylvinyléther ou un ester allylique ou méthallylique, come décrits dans la demande FR-A-2262303, dont le contenu est incorporé dans la présente demande à titre de référence.

Comme polymères filmogènes liposolubles utilisables dans l'invention, on peut également citer les polyalkylènes et notamment les copolymères d'alcènes en $C_2$-$C_{20}$ comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène liposoluble peut être présent dans la composition en une teneur allant de 0,1 % à 15 % en poids, par rapport au poids total de la composition, et mieux de 2 % à 10 % en poids.

La composition comprend au moins une matière colorante comme les composés pulvérulents et/ou les colorants liposolubles, par exemple à raison de 0,01 à 50 % du poids total de la composition. Les composés pulvérulents peuvent être choisis parmi les pigments et/ou les nacres habituellement utilisés dans les compositions cosmétiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition peut également comprendre des charges qui peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de Nylon (Orgasol de chez Atochem), de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut comprendre, en outre, un agent épaississant de la phase grasse liquide. L'agent épaississant peut être choisi parmi les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX.

On peut également employer des silices traitées, des gommes de guar alkylées liposolubles.

L'agent épaississant peut être présent en une teneur permettant d'ajuster la viscosité voulue. Il peut notamment âtre présent en une teneur allant de 0,1 % à 10 % en poids, par rapport au poids total de la composition.

La composition peut contenir, en outre, tout additif usuellement utilisés dans de telles compositions tels que les parfums, les conservateurs, les tensioactifs, les plastifiants, les sequestrants, les vitamines, les protéines, les céramides, les agents alcalinisants ou acidifiants, les émollients.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Selon un mode de réalisation de l'invention, la composition peut être avantageusement anhydre, et peut contenir moins de 10 % d'eau par rapport au poids total de la composition. Elle peut alors se présenter sous forme de gel huileux ou de pâte.

L'invention est illustrée plus en détail dans les exemples suivants.

La viscosité de chaque composition a été mesurée à 25 °C, à l'aide du viscosimètre RHEOMATT 180 vendu par la société RHEO, équipé du mobile n° 3 ou 4, à une vitesse de cisaillement de 200 s$^{-1}$, la mesure étant effectuée après 10 minutes de rotation du mobile.

### Exemple 1 de dispersion de polymère :

On prépare une dispersion de copolymère non réticulé d'acrylate de méthyle et d'acide acrylique dans un rapport 85/15, dans de l'isododécane, selon la méthode de l'exemple 7 du document EP-A-749 747. On obtient ainsi une dispersion de particules de poly(acrylate de méthyle/acide acrylique) stabilisées en surface dans de l'isododécane par un copolymère dibloc séquencé polystyrène/copoly(éhylènepropylène) vendu sous le nom de KRATON G1701 (Shell), ayant un taux de matière sèche de 22,6% en poids et une taille moyenne des particules de 175 nm (polydispersité : 0,05) et une Tg de 20°C. Ce copolymère est filmifiable.

### Exemples 2 à 4 de mascara (comparatif) :

On a préparé 3 compositions 2 à 4 suivantes ; la composition 2 ne contenant que 1,13 % en matière sèche du polymère de la dispersion de l'exemple 1 ne fait pas partie de l'invention.

- Dispersion de polymère de l'exemple 1      x g
- Paraffine      2,2 g
- Cire de carnauba      4,5 g
- Cire d'abeille      8,3 g
- Copolymère acétate de vinyle/stéarate d'allyle (65/35) (Mexomère PQ de CHIMEX)      2,2 g
- Polylaurate de vinyle (Mexomère PP de CHIMEX)      0,75 g
- Amidon de riz      0,85 g
- Bentonite      5,32 g
- Carbonate de propylène      1,74 g
- Pigments      4,6 g
- Conservateurs      qs
- Isododécane      qsp      100 g

Pour chaque composition, on a étalé un film de 300 μm d'épaisseur (avant séchage) sur une plaque de verre puis après séchage pendant 24 heures à 30 °C, on a frotté le film avec un coton tige imbibé d'eau ou du démaquillant Bifacil de chez Lancôme. On a compté le nombre de passage de coton tige à effectuer pour endommager le film.

On a obtenu les résultats suivants :

| Exemple | 2 (HI) | 3 | 4 |
|---|---|---|---|
| X (MS*) | 5 (1,13) | 10 (2,23) | 30 (6,78) |
| Viscosité en Pa.s (en poise) | 5,9 (59) mobile 4 | 6,2 (62) mobile 4 | 13 (130) mobile 4 |
| **Nombre de passage** *Eau* *Bifacil* | 8 13 | 12 15 | 20 42 |
| HI : Hors invention MS *: teneur en gramme exprimée en matière sèche de particules de polymère de la dispersion de l'exemple 1. | | | |

On constate que les compositions des exemples 3 à 4 selon l'invention permettent d'obtenir un film parfaitement résistant aux frottements en présence d'eau, alors que la composition de l'exemple 2 ne faisant pas partie de l'invention conduit à un film qui résiste moins aux frottements.

**Exemple 7 de dispersion de polymère :**

On prépare une dispersion de copolymère non réticulé de méthacrylate de méthyle et d'acide acrylique dans un rapport 85/15, dans de l'isododécane, selon la méthode de l'exemple 1 du document EP-A-749 746, en remplaçant l'heptane par de l'isododécane. On obtient ainsi une dispersion de particules de poly(méthacrylate de méthyle/acide acrylique) stabilisées en surface dans de l'isododécane par un copolymère dibloc séquencé polystyrène/copoly(éhylènepropylène) vendu sous le nom de KRATON G1701 (Shell), ayant un taux de matière sèche de 25,5 % en poids et une taille moyenne des particules de 150 nm (polydispersité < 0,1) et une Tg de 100 °C. Ce copolymère est non filmifiable.

**Exemple 8 de mascara :**

On a préparé un mascara ayant la composition décrite à l'exemple 3 en utilisant 10 g de la dispersion de polymère de l'exemple 6, soit 2,55 g en matières sèches. La composition a une viscosité, mesurée au mobile n° 4, de 7,8 Pa.s (78 poises). On a testé cette composition selon le test décrit aux exemples 2 à 5. On a constaté que le film est endommagé après 50 passages du cotton tige imprégné d'eau, et de 40 passages du cotton tige imprégné du démaquillant Bifacil. La composition de l'exemple 6 permet donc d'obtenir un film parfaitement résistant aux frottements.

La compositon s'applique facilement sur les cils et permet d'obtenir un maquillage résistant aux frottements, notamment des doigts.

**Revendications**

1.  Mascara comprenant, dans un milieu physiologiquement acceptable, une dispersion de particules de polymère stabilisées en surface dans une phase grasse liquide, **caractérisée par le fait qu'**elle comprend au moins 2 % en poids, par rapport au poids total de la composition, desdites particules de polymère, au moins une matière colorante, et que la composition a une viscosité, mesurée à 25 °C, à un cisaillement de 200 s$^{-1}$, allant de 2 Pa.s à 17 Pa.s.

2.  Mascara selon la revendication 1, **caractérisé par le fait que** la viscosité va de 5 Pa.s à 13 Pa.s.

3.  Mascara selon l'une quelconque des revendications précédentes, **caractérisé par le fait que** le polymère est choisi parmi les polymères radicalaires, les polycondensats, les polymères d'origine naturelle et leurs mélanges.

4.  Mascara selon l'une quelconque des revendications précédentes, **caractérisé par le fait que** le polymère est choisi parmi les polyuréthannes, polyuréthannes-acryliques, polyurées, polyurée/polyuréthannes, polyester-polyuréthannes, polyéther-polyuréthannes, polyesters, polyesters amides, polyesters à chaîne grasse, alkydes ; polymères ou copolymères acryliques et/ou vinyliques ; polymères siliconés, polymères fluorés et leurs mélanges.

5.  Mascara selon l'une quelconque des revendications précédentes, **caractérisé par le fait que** le polymère est un polymère filmifiable.

6.  Mascara selon l'une quelconque des revendications précédentes, **caractérisé par le fait que** les particules de polymères sont stabilisées par un stabilisant choisi parmi les polymères séquencés, les polymères greffés, les polymères statistiques et leurs mélanges.

7.  Mascara selon la revendication 6, dans lequel le stabilisant est choisi parmi les polymères siliconés greffés avec une chaîne hydrocarbonée ; les polymères hydrocarbonés greffés avec une chaîne siliconée ; les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins d'un polyéther ; les copolymères de (méth)acrylates d'alkyl en C$_1$-C$_4$ et de (méth)acryla-tes d'alkyl en C$_8$-C$_{30}$ ; les copolymères blocs greffés ou séquencés comprenant au moins un bloc résultant de la polymérisation de monomères éthyléniques comportant une ou plusieurs liaisons éthylénique éventuellement conjuguées et au moins un bloc d'un polymère styrénique ; les copolymères blocs greffés ou séquencés comprenant au moins un bloc résultant de la polymérisation de monomères éthyléniques et au moins un bloc d'un polymère acrylique ; les copolymères blocs greffés ou séquencés comprenant au moins un bloc résultant de la polymérisation de monomère éthylénique et au moins un bloc d'un polyéther.

8. Mascara selon la revendication 6 ou 7, **caractérisé en ce que** le stabilisant est un polymère bloc greffé ou séquencé, comprenant au moins un bloc résultant de la polymérisation de monomères éthyléniques comportant une ou plusieurs liaisons éthylénique éventuellement conjuguées et au moins un bloc d'un polymère styrénique.

9. Mascara selon l'une quelconque des revendications précédentes, **caractérisé par le fait que** les particules de polymères sont présentes en une teneur en matière sèche allant de 2 % à 50 % en poids, par rapport au poids total de la composition, de préférence de 4 % à 40 % en poids, et mieux de 5 % à 30 % en poids.

10. Mascara selon l'une quelconque des revendications précédentes, **caractérisé par le fait que** la phase grasse liquide est constituée d'huiles d'origine minérale, animale, végétale ou synthétique, hydrocarbonées, fluorées et/ou siliconées, seules ou en mélange.

11. Mascara selon l'une des revendications précédentes, dans lequel la phase grasse liquide est choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées ; les huiles volatiles telles que l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en $C_8$-$C_{16}$, et notamment l'isododécane.

12. Mascara selon l'une quelconque des revendications précédentes, **caractérisé par le fait que** la phase grasse liquide est choisie dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2,}$
- ou leurs mélanges.

13. Mascara selon l'une quelconque des revendications précédentes, **caractérisé en ce que** la phase grasse contient au moins une huile volatile à température ambiante.

14. Mascara selon l'une quelconque des revendications précédentes, **caractérisé par le fait que** la composition comprend au moins un additif choisi dans le groupe formé par les parfums, les conservateurs, les tensioactifs, les plastifiants, les sequestrants, les vitamines, les protéines, les céramides, les agents alcalinisants ou acidifiants, les émollients, les charges.

15. Mascara selon l'une quelconque des revendications précédentes, **caractérisé par le fait que** la composition est anhydre.

16. Mascara selon l'une quelconque des revendications précédentes, **caractérisé par le fait que** la composition est une composition de maquillage, une base de maquillage, une composition à appliquer sur un maquillage, une composition de traitement cosmétique des fibres kératiniques.

17. Procédé de revêtement des cils, **caractérisé par le fait que** l'on applique sur les cils un mascara selon l'une quelconque des revendications 1 à 16.

18. Utilisation d'un mascara tel que défini selon l'une quelconque des revendications 1 à 16 pour l'obtention d'un film déposé sur les cils, résistant aux frottements.

**Patentansprüche**

1. Mascara, die in einem physiologisch akzeptablen Medium eine Dispersion von an der Oberfläche stabilisierten Polymerpartikeln in einer flüssigen Fettphase enthält, **dadurch gekennzeichnet, dass** sie, bezogen auf das Gesamtgewicht der Zusammensetzung, mindestens 2 Gew.-% dieser Polymerpartikel und mindestens ein Farbmittel enthält und dadurch, dass die Zusammensetzung eine bei 25 °C bei einer Schergeschwindigkeit von 200 s$^{-1}$ gemessene Viskosität von 2 bis 17 Pa·s aufweist.

2. Mascara nach Anspruch 1, **dadurch gekennzeichnet, dass** die Viskosität im Bereich von 5 bis 13 Pa·s liegt.

3. Mascara nach einem der vorhergehenden Ansprüche, **dadurch gekennzeichnet, dass** das Polymer unter den radikalischen Polymeren, Polykondensaten und Polymeren natürlicher Herkunft und deren Gemischen ausgewählt ist.

4. Mascara nach einem der vorhergehenden Ansprüche, **dadurch gekennzeichnet, dass** das Polymer unter den Polyurethanen, Polyurethan-Acrylpolymeren, Polyharnstoffen, Polyharnstoff-Polyurethanen, Polyester-Polyurethanen, Polyether-Polyurethanen, Polyestern, Polyesteramiden, Polyestern mit Fettkette, Alkydpolymeren; Acrylpolymeren, Acrylcopolymeren und/ oder Vinylpolymeren oder Vinylcopolymeren; Siliconpolymeren, fluorierten Polymeren und deren Gemischen ausgewählt ist.

5. Mascara nach einem der vorhergehenden Ansprüche, **dadurch gekennzeichnet, dass** das Polymer ein filmbildendes Polymer ist.

6. Mascara nach einem der vorhergehenden Ansprüche, **dadurch gekennzeichnet, dass** die Polymerpartikel durch ein Stabilisierungsmittel stabilisiert sind, das unter den sequentiellen Polymeren, gepfropften Polymeren, statistischen Polymeren und deren Gemischen ausgewählt ist.

7. Mascara nach Anspruch 6, wobei das Stabilisierungsmittel unter den mit einer Kohlenwasserstoffkette gepfropften Siliconpolymeren; mit einer Siliconkette gepfropften Kohlenwasserstoffpolymeren; sequentiellen oder gepfropften Blockcopolymeren, die mindestens einen Block vom Polyorganosiloxantyp und mindestens einen Block eines radikalischen Polymers enthalten; sequentiellen oder gepfropften Blockcopolymeren, die mindestens einen Block vom Polyorganosiloxantyp und mindestens einen Polyetherblock enthalten; Copolymeren von C$_{1-4}$-Alkyl(meth) acrylaten und C$_{8-30}$-Alkyl(meth)acrylaten; sequentiellen oder gepfropften Blockcopolymeren, die mindestens einen Block, der bei der Polymerisation von ethylenischen Monomeren entsteht, die eine oder mehrere ethylenische Doppelbindungen enthalten, die gegebenenfalls konjugiert sind, und mindestens einen Block eines Styrolpolymers enthalten; sequentiellen oder gepfropften Blockcopolymeren, die mindestens einen Block, der bei der Polymerisation von ethylenischen Monomeren entsteht, und mindestens einen Block eines Acrylpolymers enthalten; sequentiellen oder gepfropften Blockcopolymeren, die mindestens einen Block, der bei der Polymerisation von ethylenischen Monomeren entsteht, und mindestens einen Polyetherblock enthalten, ausgewählt ist.

8. Mascara nach Anspruch 6 oder 7, **dadurch gekennzeichnet, dass** das Stabilisierungsmittel ein sequentielles oder gepfropftes Blockpolymer ist, das mindestens einen Block, der bei der Polymerisation von ethylenischen Monomeren, die eine oder mehrere ethylenische, gegebenenfalls konjugierte Doppelbindungen enthalten, gebildet wird, und mindestens einen Block eines Styrolpolymers enthält.

9. Mascara nach einem der vorhergehenden Ansprüche, **dadurch gekennzeichnet, dass** die Polymerpartikel in einem Trockensubstanzgehalt von 2 bis 50 Gew.-%, bezogen auf das Gesamtgewicht der Zusammensetzung, vorzugsweise 4 bis 40 Gew.-% und besser 5 bis 30 Gew.-% enthalten sind.

10. Mascara nach einem der vorhergehenden Ansprüche, **dadurch gekennzeichnet, dass** die flüssige Fettphase aus Ölen mineralischer, tierischer, pflanzlicher oder synthetischer Herkunft, Kohlenwasserstoffölen, fluorierten Ölen und/oder Siliconölen und/oder deren Gemischen besteht.

11. Mascara nach einem der vorhergehenden Ansprüche, wobei die flüssige Fettphase ausgewählt ist unter: Paraffinöl oder Vaselineöl, Nerzöl, Schildkrötenöl, Sojaöl, Perhydrosqualen, Süßmandelöl, Calophyllumöl, Palmöl, Parleam, Traubenkernöl, Sesamöl, Maisöl, Rapsöl, Sonnenblumenöl, Bauwollsamenöl, Aprikosenkernöl, Ricinusöl, Avocadoöl, Jojobaöl, Olivenöl oder Getreidekeimöl; Estern von Lanolinsäure, Ölsäure, Laurinsäure, Stearinsäure; Fettsäureestern, wie Isopropylmyristat, Isopropylpalmitat, Butylstearat, Hexyllaurat, Diisopropyladipat, Isononylisononat, 2-Ethylhexylpalmitat, 2-Hexyldecyllaurat, 2-Octyldecylpalmitat, 2-Octyldodecylmyristat, 2-Octyldodecyllactat, 2-Diethylhexylsuccinat, Diisostearylmalat, Glyceryltriisostearat oder Diglyceryltriisostearat; höheren Fettsäuren, wie Myristinsäure, Palmitinsäure, Stearinsäure, Behensäure, Ölsäure, Linolsäure, Linolensäure oder Isostearinsäure; höheren Fettalkoholen, wie Cetanol, Stearylalkohol oder Oleylalkohol, Linoleylalkohol, Linolenylalkohol, Isostearylalkohol oder Octyldodecanol; Siliconölen, wie PDMS, die gegebenenfalls phenyliert sind, wie Phenyltrimethicone, oder gegebenenfalls mit aliphatischen und/oder aromatischen Gruppen oder mit funktionellen Gruppen, wie Hydroxy-, Thiol- und/oder Amingruppen substituiert sind; mit Fettsäuren, Fettalkoholen oder mit Polyoxyalkylenen modifizierte Polysiloxanen, fluorierten Siliconen, perfluorierten Ölen; flüchtigen Ölen, wie Octamethylcyclotetrasiloxan, Decamethylcyclopentasiloxan, Hexadecamethylcyclohexasiloxan, Heptamethylhexyltrisiloxan und Heptamethyloctyltrisiloxan oder $C_{8-16}$-Isoparaffinen, insbesondere Isododecan.

**12.** Mascara nach einem der vorhergehenden Ansprüche, **dadurch gekennzeichnet, dass** die flüssige Fettphase unter den folgenden Verbindungen ausgewählt ist:

- den flüssigen nicht wässrigen Fettphasen mit einem Gesamtsolubilitätsparameter gemäß den Solubilitätsraum nach HANSEN unter 17 $(MPa)^{\frac{1}{2}}$,
- den Monoalkoholen mit einem Gesamtsolubilitätsparameter nach dem Solubilitätsraum von HANSEN von höchstens 20 $(MPa)^{\frac{1}{2}}$,
- oder ihren Gemischen.

**13.** Mascara nach einem der vorhergehenden Ansprüche, **dadurch gekennzeichnet, dass** die Fettphase mindestens ein bei Umgebungstemperatur flüchtiges Öl enthält.

**14.** Mascara nach einem der vorhergehenden Ansprüche, **dadurch gekennzeichnet, dass** die Zusammensetzung mindestens einen Zusatzstoff enthält, der unter den Parfums, Konservierungsmitteln, grenzflächenaktiven Stoffen, Weichmachern, Maskierungsmitteln, Vitaminen, Proteinen, Ceramiden, Alkalisierungsmitteln, Ansäuerungsmitteln, Emollientien und Füllstoffen ausgewählt ist.

**15.** Mascara nach einem der vorhergehenden Ansprüche, **dadurch gekennzeichnet, dass** die Zusammensetzung wasserfrei ist.

**16.** Mascara nach einem der vorhergehenden Ansprüche, **dadurch gekennzeichnet, dass** es sich bei der Zusammensetzung um eine Zusammensetzung zum Schminken, Grundmasse zum Schminken, Zusammensetzung, die auf eine Schminke aufgetragen wird oder eine Zusammensetzung zur kosmetischen Behandlung von Keratinfasern handelt.

**17.** Verfahren zur Beschichtung von Wimpern, **dadurch gekennzeichnet, dass** auf die Wimpern eine Mascara nach einem der Ansprüche 1 bis 16 aufgetragen wird.

**18.** Verwendung einer Mascara nach einem der Ansprüche 1 bis 16 zur Erzeugung eines auf den Wimpern abgeschiedenen Films, der gegenüber Reiben beständig ist.

**Claims**

**1.** Mascara comprising, in a physiologically acceptable medium, a dispersion of surface-stabilized polymer particles in a liquid fatty phase, **characterized in that** it comprises at least 2% by weight, relative to the total weight of the composition, of the said polymer particles and at least one colourant, and **in that** the composition has a viscosity, measured at 25°C, at a shear rate of 200 s$^{-1}$, ranging from 2 Pa.s to 17 Pa.s.

**2.** Mascara according to Claim 1, **characterized in that** the viscosity ranges from 5 Pa.s to 13 Pa.s.

**3.** Mascara according to either of the preceding claims, **characterized in that** the polymer is chosen from radical-mediated polymers, polycondensates and polymers of natural origin, and mixtures thereof.

4. Mascara according to any one of the preceding claims, **characterized in that** the polymer is chosen from polyurethanes, polyurethane-acrylics, polyureas, polyurea-polyurethanes, polyester-polyurethanes, polyether-polyurethanes, polyesters, polyesteramides, polyesters containing a fatty chain, alkyds; acrylic and/or vinyl polymers or copolymers; silicone polymers and fluoro polymers, and mixtures thereof.

5. Mascara according to any one of the preceding claims, **characterized in that** the polymer is a film-forming polymer.

6. Mascara according to any one of the preceding claims, **characterized in that** the polymer particles are stabilized with a stabilizer chosen from block polymers, grafted polymers and random polymers, and mixtures thereof.

7. Mascara according to Claim 6, in which the stabilizer is chosen from silicone polymers grafted with a hydrocarbon-based chain; hydrocarbon-based polymers grafted with a silicone chain; grafted-block or block copolymers comprising at least one block of polyorganosiloxane type and at least one block of a radical-mediated polymer; grafted-block or block copolymers comprising at least one block of polyorganosiloxane type and at least one block of a polyether; copolymers of $C_1$-$C_4$ alkyl (meth)acrylates and of $C_8$-$C_{30}$ alkyl (meth)acrylates; grafted-block or block copolymers comprising at least one block resulting from the polymerization of ethylenic monomers comprising one or more optionally conjugated ethylenic bonds and at least one block of a styrene polymer; grafted-block or block copolymers comprising at least one block resulting from the polymerization of ethylenic monomers and at least one block of an acrylic polymer; grafted-block or block copolymers comprising at least one block resulting from the polymerization of an ethylenic monomer and at least one block of a polyether.

8. Mascara according to Claim 6 or 7, **characterized in that** the stabilizer is a grafted-block or block polymer comprising at least one block resulting from the polymerization of ethylenic monomers comprising one or more optionally conjugated ethylenic bonds and at least one block of a styrene polymer.

9. Mascara according to any one of the preceding claims, **characterized in that** the polymer particles are present in a solids content ranging from 2% to 50% by weight, relative to the total weight of the composition, preferably from 4% to 40% by weight and better still from 5% to 30% by weight.

10. Mascara according to any one of the preceding claims, **characterized in that** the liquid fatty phase consists of oils of mineral, animal, plant or synthetic origin or hydrocarbon-based oils, fluoro oils and/or silicone oils, alone or as a mixture.

11. Mascara according to one of the preceding claims, in which the liquid fatty phase is chosen from liquid paraffin, liquid petroleum jelly, mink oil, turtle oil, soybean oil, perhydrosqualene, sweet almond oil, beauty-leaf oil, palm oil, parleam oil, grape seed oil, sesame oil, corn oil, rapeseed oil, sunflower oil, cotton oil, apricot oil, castor oil, avocado oil, jojoba oil, olive oil or cereal germ oil; esters of lanolic acid, of oleic acid, of lauric acid or of stearic acid; fatty esters such as isopropyl myristate, isopropyl palmitate, butyl stearate, hexyl laurate, diisopropyl adipate, isononyl isononate, 2-ethylhexyl palmitate, 2-hexyldecyl laurate, 2-octyldecyl palmitate, 2-octyldodecyl myristate or lactate, 2-diethylhexyl succinate, diisostearyl malate, glyceryl triisostearate or diglyceryl triisostearate; higher fatty acids such as myristic acid, palmitic acid, stearic acid, behenic acid, oleic acid, linoleic acid, linolenic acid or isostearic acid; higher fatty alcohols such as cetanol, stearyl alcohol, oleyl alcohol, linoleyl alcohol, linolenyl alcohol, isostearyl alcohol or octyldodecanol; silicone oils such as PDMSs which are optionally phenylated, such as phenyltrimethicones, or optionally substituted with aliphatic and/or aromatic groups, or optionally substituted with functional groups such as hydroxyl, thiol and/or amine groups; polysiloxanes modified with fatty acids, fatty alcohols or polyoxyalkylenes, fluorosilicones, perfluoro oils; volatile oils such as octamethylcyclotetrasiloxane, decamethylcyclopentasiloxane, hexadecamethylcyclohexasiloxane, heptamethylhexyltrisiloxane, heptamethyloctyltrisiloxane or $C_8$-$C_{16}$ isoparaffins, and in particular isododecane.

12. Mascara according to any one of the preceding claims, **characterized in that** the liquid fatty phase is chosen from the group comprising:

- non-aqueous liquid compounds having an overall solubility parameter according to the Hansen solubility space of less than 17 $(MPa)^{\frac{1}{2}}$,
- or monoalcohols having an overall solubility parameter according to the Hansen solubility space of less than or equal to 20 $(MPa)^{\frac{1}{2}}$,
- or mixtures thereof.

**13.** Mascara according to any one of the preceding claims, **characterized in that** the fatty phase contains at least one oil which is volatile at room temperature.

**14.** Mascara according to any one of the preceding claims, **characterized in that** the composition comprises at least one additive chosen from the group formed by fragrances, preserving agents, surfactants, plasticizers, sequestering agents, vitamins, proteins, ceramides, acidifying or basifying agents, emollients and fillers.

**15.** Mascara according to any one of the preceding claims, **characterized in that** the composition is anhydrous.

**16.** Mascara according to any one of the preceding claims, **characterized in that** the composition is a make-up composition, a make-up base, a composition to be applied to a make-up, or a cosmetic treatment composition for keratin fibres.

**17.** Process for coating eyelashes, **characterized in that** a mascara according to any one of Claims 1 to 16 is applied to the eyelashes.

**18.** Use of a mascara as defined according to any one of Claims 1 to 16, to obtain a film deposited on eyelashes, which withstands rubbing.